(19) Europäisches Patentamt
European Patent Office
Office européen des brevets

(11) Publication number: **0 030 392**
**B1**

(12) **EUROPEAN PATENT SPECIFICATION**

(45) Date of publication of patent specification: **16.05.84**

(51) Int. Cl.$^3$: **C 07 C 149/24** // C07C149/18, C07C149/23

(21) Application number: **80108272.8**

(22) Date of filing: **20.12.78**

(60) Publication number of the earlier application in accordance with Art. 76 EPC: **0 003 740**

(54) **Intermediates for use in the preparation of carbapenems.**

(30) Priority: **19.01.78 GB 216678**
**14.03.78 GB 998578**

(43) Date of publication of application:
**17.06.81 Bulletin 81/24**

(45) Publication of the grant of the patent:
**16.05.84 Bulletin 84/20**

(84) Designated Contracting States:
**BE CH DE FR GB NL**

(56) References cited:

**TETRAHEDRON LETTERS, no. 51, December 1972, Oxford, GB S. HOFF: "Reductive cleavage of 1,4-thiazines the generation of powerful reactive intermediates for the synthesis of heterocyclic compounds", pages 5199-5202**

(73) Proprietor: **BEECHAM GROUP PLC**
**Beecham House Great West Road**
**Brentford Middlesex (GB)**

(72) Inventor: **Ponsford, Roger John**
**5 Copse Close Wimblehurst Park**
**Horsham Sussex (GB)**
Inventor: **Baxter, Andrew John Gilby**
**3 Albina Garth Medon**
**Hull N. Humberside (GB)**
Inventor: **Southgate, Robert**
**7 Tillets Lane**
**Warnham West Sussex (GB)**
Inventor: **Roberts, Patricia Margaret**
**15 Bakehouse Road**
**Horley Surrey (GB)**

(74) Representative: **Hesketh, Alan, Dr. et al**
**European Patent Attorney Beecham Pharmaceuticals Great Burgh Yew Tree Bottom Road**
**Epsom, Surrey, KT18 5XQ (GB)**

**EP 0 030 392 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This invention relates to a class of compounds, which are useful as intermediates in the production of 3-substituted carbapenems.

British Patent No. 1,483,142 discloses that the compound of the formula (I):

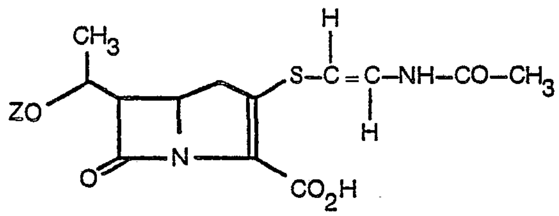

(I)

wherein Z is $HO_3S$ and its salts may be obtained by fermentation of strains of *Streptomyces olivaceus.* Danish Patent Application No. 984/78 discloses that the compound of the formula (I) wherein Z is H and its salts could also be obtained by fermentation of strains of that organism. We have found that a distinct class of synthetic antibacterial agents which contain a $\beta$-lactam ring fused to a pyrroline ring may be prepared.

In our co-pending European Patent Application No: 78300873.3 we have disclosed and claimed the novel compounds of the formula (II):

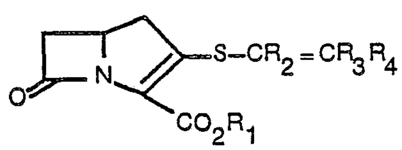

(II)

wherein $R_1$ is a hydrogen atom, a salt forming cation or an esterifying group; $R_2$ is a hydrogen atom or a lower alkyl group; $R_3$ is a hydrogen atom or a lower alkyl group; and $R_4$ is a hydrogen atom, lower alkyl group, phenyl group or a phenyl group substituted by one or two groups selected from fluorine, chlorine, $OR_5$, $NH.CO.R_5$, $NH.CO_2R_5$ or $CO_2R_5$, where $R_5$ is a lower alkyl or benzyl group, or $R_4$ is a $CO_2R_5$ where $R_5$ is a lower alkyl or benzyl group or $R_4$ is a $NH.CO_nR_6$ group where $R_6$ is a lower alkyl group, a phenyl group or a phenyl group substituted by one or two halogen atoms, lower alkyl or lower alkoxyl groups; and n is 1 or 2.

When used herein the term "lower" means the group contains up to 4 carbon atoms.

A reaction sequence leading to the compounds of the formula (II) is as follows, wherein $R_1$ is an esterifying group.

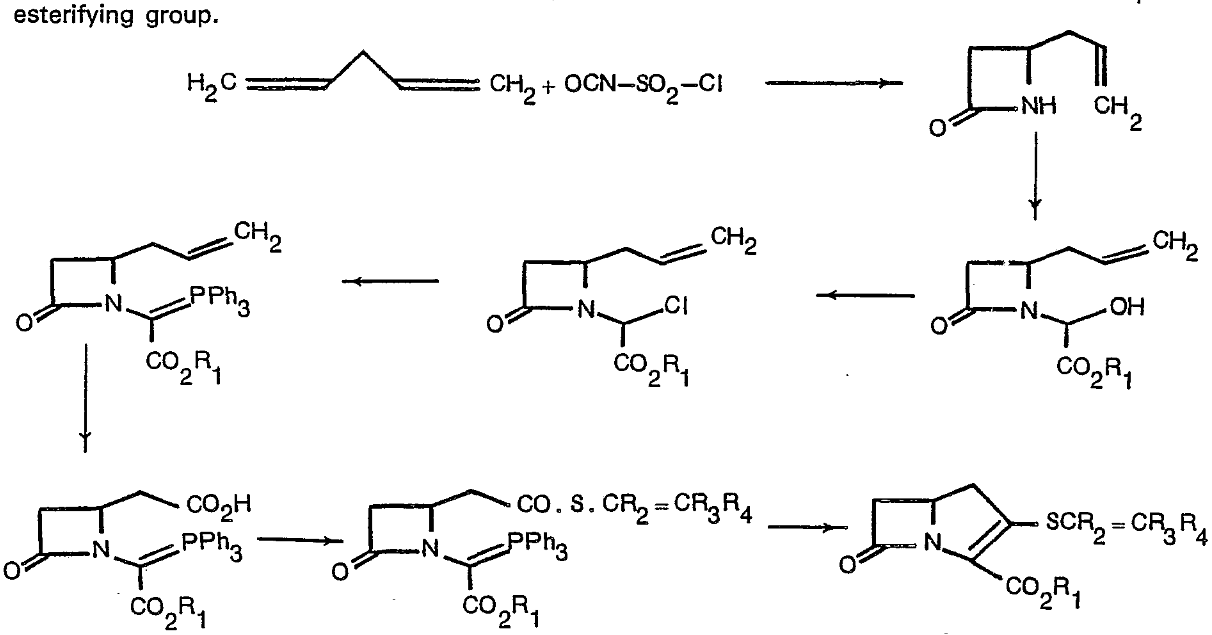

The process for the preparation of the compounds of the formula (II) comprises the ring closing elimination of the elements of triphenylphosphine oxide from a compound of the formula (III):

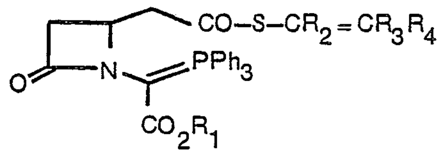

(III)

wherein $R_1$ is an esterifying group, and $R_2$, $R_3$ and $R_4$ are as defined in relation to formula (II); and thereafter cleaving the ester to yield the carboxylic acid or its salt.

The compound of the formula (III) may be prepared by the reaction of a corresponding compound of the formula (IV):

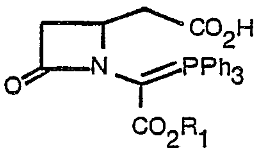

(IV)

wherein $R_1$ is as defined in relation to formula (III) with a diloweralkylphosphorochloridate and a triloweralkylamine followed by reaction with a thallium, silver, sodium or lithium salt of the compound of the formula (V):

$$H—S—CR_2{=}CR_3R_4 \qquad (V)$$

wherein $R_2$, $R_3$ and $R_4$ are as defined in relation to formula (II).

Certain compounds of the formula (V) are novel. Accordingly, the present invention provides the salts of the compounds of the formula (I) wherein $R_2$ and $R_3$ are hydrogen and $R_4$ is a $NH.CO_nR_6$ group as defined in relation to formula (II).

Preferred compounds of the formula (V) are those wherein n is 1 and $R_6$ is methyl. Suitable salts of these compounds include the sodium, thallium, silver and lithium salts.

The following Examples illustrate the invention. The use of the compounds of this invention in the preparation of bicyclic carbapenems is exemplified in European Patent Application No. 78300873.3.

## Example 1

Silver Z-2-acetamido-1-ethenylthiolate

(i) Preparation of Tritylthioacetaldehydediethylacetal.

$$\underset{(1)}{TrSH} + \underset{(2)}{BrCH_2CH(OEt)_2} \longrightarrow \underset{(3)}{TrSCH_2CH(OEt)_2}$$

Sodium (0.46g) was dissolved in absolute alcohol (50ml) and tritylthiol (1) (5.52g) was added followed by bromoethyldiethylacetal (2) (4g). The mixture was refluxed for three hours, filtered and the solvent evaporated. The residue was re-evaporated from toluene and chromatographed on Merck Keiselgel 60 (<230 mesh) using petrol (60—80°)/ethyl acetate as eluant. The product (3) was obtained as an almost colourless oil (6.5 g; 83%), $\upsilon_{max}$ ($CHCl_3$) 1600, 1495, 1450, 1120, 1060 $cm^{-1}$. $\delta$ *ppm* ($CDCl_3$) 1.16 (3H, t, J8Hz, $CH_3$), 2.51 (2H, d, J 6Hz, $CH_2CH$), 3.44 (4H, m, $CH_2$'s), 4.18 (1H, t, 6Hz, $CH_2CH$), 7.40 (15H, m, *Tr*).

(ii) Preparation of Z-2-Acetamido-1-trityl-thioethene

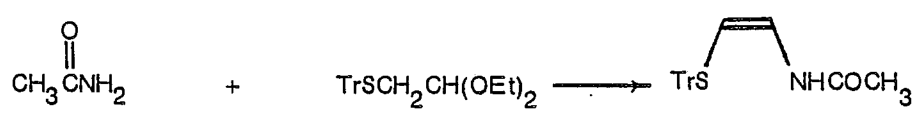

(4) (3) (5)

Tritylthioacetaldehydediethylacetal (3) (5g) was dissolved in benzene (100ml) and acetamide (4) (4g) was added. The mixture was heated with stirring to dissolve as much of the acetamide as possible. The mixture was cooled and p.t.s.a. monohydrate (1.5g) was added. The reaction mixture was then heated under reflux with removal of water for 1½ hours. The solvent was evaporated and the residue chromatographed on Merck Keiselgel 60 (<230 mesh) to yield Z-2-acetamido-1-tritylthioethene (5) as a white crystalline solid (400mg) from ethyl acetate/petrol (60—80°) m.p. 154° $\upsilon_{max}$ ($CHCl_3$) 3400, 1695, 1625, 1260 $cm^{-1}$. $\delta$ *ppm* [$(CD_3)_2CO$] 1.97 (3H, s, $COCH_3$) 4.90 (1H, d, J 8Hz, $TrSCH{=}$), 6.88 (1H, dd, J 11, 8Hz, $NHCH{=}$, collapses to a doublet (J 8Hz) on $D_2O$ exchange), 7.32 (15H, s, *Tr*), 2.45 (1H, br. signal, NH [exchangeable]), $\lambda_{max}$ (EtOH) 262 nm. (*Found:* C, 76.50; H, 5.84; N, 3.76, $C_{23}H_{21}NOS$ requires C, 76.88; H, 5.85; N, 3.90%).

(iii) Preparation of Silver Z-2-Acetamido-1-ethenylthiolate.

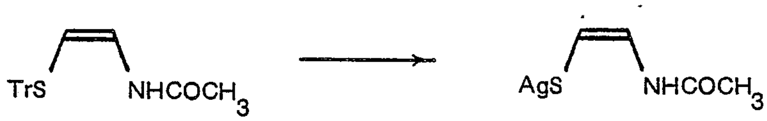

(5) (6)

Z-2-Acetamido-1-triphenylmethylthioethene (5) (72 mg) was dissolved in methanol (10 ml) and silver nitrate (34mg) in methanol (2ml) was added together with pyridine (16mg). The mixture was stirred at RT overnight and the yellow precipitate collected by centrifugation. It was washed once with methanol and once with ether and dried to give (6) as a yellow powder (41mg). $\upsilon_{max}$ (nujol mull) 3300, 1675, 1625cm$^{-1}$.

## Example 2

Silver E-2-acetamido-1-ethenylthiolate

(i) Preparation of Z-2-Acetamidoethenylthioacetate.

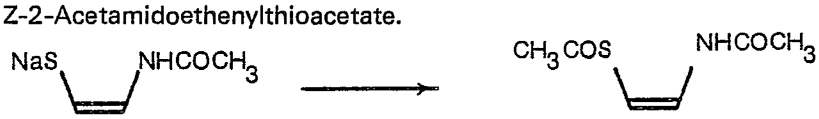

(7) (8)

To acetic anhydride (0.62g) in dry tetrahydrofuran (50ml) at 0° under argon was added the sodium thiolate (7) (1.2g, prepared as in example 5 (iii)). The mixture was stirred for 16 hours, filtered and the solvent was removed under reduced pressure. Chromatography on silica eluting with chloroform/ethanol mixtures gave the *cis*-S-acetate (8) (0.57g) as needles m.p. 110—114° (benzene) having $\lambda_{max}$ (EtOH) 260 ($\varepsilon$ 12,600) nm; $\upsilon_{max}$ ($CHCl_3$) 3400, 2950, 1700, 1635, 1470, 1255 and 1120 cm$^{-1}$; $\delta$ ($CDCl_3$) 2.12 (3H, s, $NCOCH_3$), 2.40 (3H, s, $SCOCH_3$), 5.65 (1H, d, J 8Hz=CH—S), 7.25 (1H, dd, J 8, 11Hz), and 7.70 (1H, br, NH). (Found: C, 45.55; H, 5.65; N, 8.65. $C_6H_9NO_2S$ requires C, 45.3; H, 5.65; N, 8.8%).

(ii) Preparation of E-2-Acetamidoethenylthioacetate

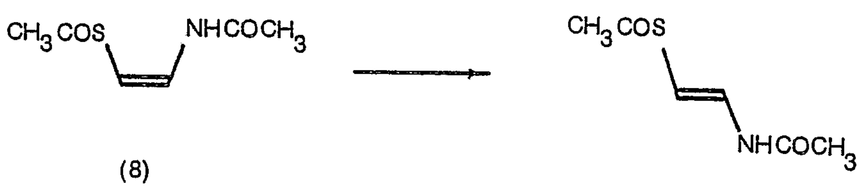

(8) (9)

To the ester (8) (0.5g) in benzene (5ml) at 50° was added diazobicycloundecane (3 drops). After heating for 3 hours under argon, the mixture was cooled, *cis*-Amide (8) (0.11g) crystallised out, and the solution obtained after filtration was chromatographed on silica eluting with chloroform/ethanol mixtures to give *cis*-amide (8) (0.135g) and *trans*-amide (9) (0.135g) m.p. 136—140° (benzene-pet. ether) having $\lambda_{max}$ (EtOH) 259 ($\varepsilon$ 12,000) nm; $\upsilon_{max-1}$; ($CHCl_3$) 3350, 3000, 1695, 1635, 1490, 1265 and 1120cm $\delta$ ($CDCl_3$) 2.10 (3H, s, $CH_3CON$), 2.37 (3H, s, $CH_3COS$), 6.0 (1H, d, J 14Hz, S—CH=), 7.23 (1H, dd, J 11, 14Hz, N—CH=), and 4.43 (1H, br, NH). (Found: C, 45.5; H, 5.80; N, 8.75. $C_6H_9NO_2S$ requires C, 45.3; H, 5.65; N, 8.8%).

(iii) Preparation of silver E-2-acetamido-1-ethenylthiolate.

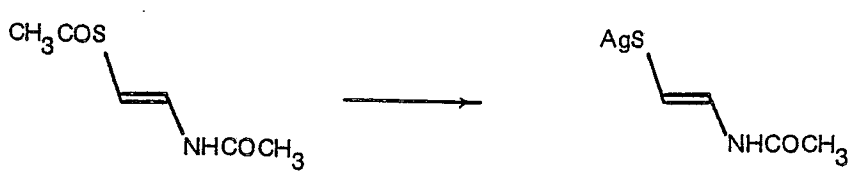

(9) (10)

To the *trans*-amide (9) (1.0g) in methanol (20ml) was added a solution of silver nitrate (1.16g) and pyridine (0.5ml) in methanol (50ml). After stirring for 18 hours the mixture was centrifuged and the solid washed once with methanol and once with ether and then dried under vacuum to give the silver thiolate (10) as an orange solid having $\nu_{max}$ (mull) 3350, 1665 and 1635 $cm^{-1}$.

Example 3

Silver Z-2-methoxycarbonylamino-1-ethenylthiolate

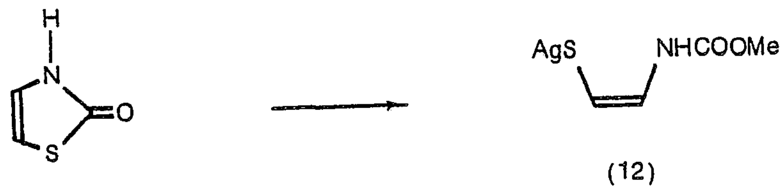

(11) (12)

To 2(3H)-thiazolone (11) (0.5g) (prepared by the method of R. Dahlbom, S. Gronowitz and B. Mathiasson, Acta Chem. Scand. *17*, 2479 (1963)) in dry methanol (5 ml) was added to a solution of silver nitrate (0.85 g) and pyridine (0.4 ml) in methanol (37.5 ml). After stirring for 18 hours the solution was centrifuged and the solid was washed with methanol and then with ether to give the thiolate (12) (0.25 g). More thiolate was obtained from the methanol solution. The methanol was removed under vacuum and the solid obtained was washed with ether (30 ml) and then with water (30 ml). The resulting solid was filtered off and dried in vacuo to give thiolate (12) (0.62 g). This gave a total yield of 0.87 g of thiolate (12) having $_{max}$ (KBr) 3400, 1705, 1630, 1515 and 1385 $cm^{-1}$.

Example 4

Silver E-2-acetamido-1-ethenylthiolate

(i) Preparation of E-2-Acetamido-1-tritylthioethene.

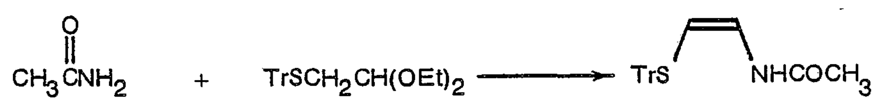

(4) (3) + (5)

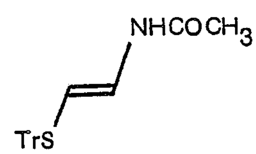

(13)

Tritylthioacetaldehydediethylacetal (3) (15.7g), acetamide (11.8g) and p.t.s.a. monohydrate (7.6g) were dissolved in DMF (30ml) and heated at 90° for three hours. The solvent was evaporated under high vacuum and re-evaporated from toluene. The residue was chromatographed on Merck Keiselgel 60 (<230 mesh) to yield Z-2-acetamido-1-tritylthioethene (5) (3g) and E-2-acetamido-1-tritylthioethene (13) (4g) as a white solid by trituration with ethyl acetate. m.p. 192—4° $\nu_{max}$ ($CHCl_3$) 1695, 1625, 1490, 1260 $cm^{-1}$ $\delta ppm$ [$(CH_{3})_{2}CO$] 1.83 (3H, s, $COCH_3$), 5.24 (1H, d, J15Hz, TrS*CH*=), 6.84 (1H, dd, J15, 9.5Hz, NH*CH*=, collapses to a doublet (J15Hz) on $D_2O$ exchange), 7.27 (15H, s, *Tr*), 10.02 (1H, d, J9.5Hz, NH [exchangeable]). (*Found*: C, 76.90; H, 6.14; N, 3.95, $C_{23}H_{21}NOS$ requires C, 76.88; H, 5.85; N, 3.90%).

(ii) Preparation of Silver E-2-Acetamido-1-ethenylthiolate (Alternative method to Example 2 (iii))

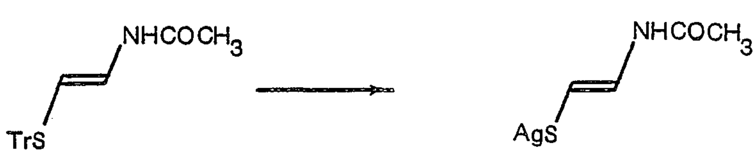

(13) (10)

E-2-Acetamido-1-tritylthioethene (13) (1.44g) was dissolved in methanol (100ml) by refluxing for 15 minutes. Silver nitrate (0.68g) in methanol (20ml) was added together with pyridine (0.32g) at RT and stirring continued for three hours. The precipitate was collected by centrifugation and washed twice with methanol and once with ether to yield (10) as a dark brown powder (0.425g) $v_{max}$ (nujol mull) 3220, 1655, 1625 $cm^{-1}$.

Example 5

Sodium Z-2-acetamido-1-ethenylthiolate

(i) Preparation of 2-(2,2′-Diethoxyethylthio)-acetamide

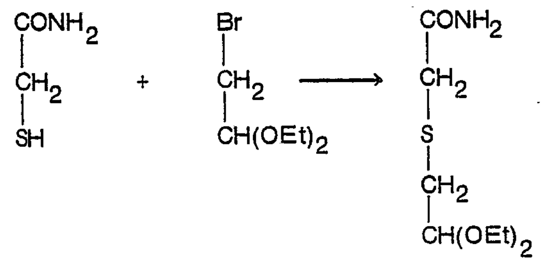

(14) (2) (15)

Sodium (0.878g) was dissolved in absolute ethanol (150ml) under an inert atmosphere. Thioglycolamide (14) (3.47g, prepared as described in Sokol and Ritter, J. Amer. Chem. Soc., *70,* 3517, 1948) was added and, when practically all the amide had dissolved, bromoacetaldehyde diethyl acetal (2) (11.5ml) was dropped in. The mixture was heated at 60—65° for 7 hours and then cooled. The sodium chloride was filtered off and the ethanol was removed under reduced pressure. The residue was dissolved in ethyl acetate (100ml) and brine (50ml) and the aqueous layer extracted once more with ethyl acetate (50ml). The combined organic extracts were dried ($Na_2SO_4$) and the solvent removed under reduced pressure to yield an oil. Chromatography on silica eluting with ethyl acetate —pet. ether mixtures gave the amide (15) (6g) as an oil having $v_{max}$ ($CHCl_3$) 3500, 3400, 3000, 1690, 1125 and 1060$cm^{-1}$; $\delta$ ($CDCl_3$) 1.20 (6H, t, J 7Hz, $CH_3$), 2.80 (2H, d, J 5Hz, —$CHCH_2$—S), 3.25 (2H, s, —$SCH_2CO$), 3.58 (4H, 2 x q, J 7Hz, —$OCH_2$—), 4.62 (1H, t, J 5Hz, —$CHCH_2S$—) and 7.00 (2H, s, $CONH_2$); m/e 207 ($M^+$, 0.2%), 103 (100%, $CH(OEt)_2^+$) and 75 (30%, $CHOH(OEt)^+$); M, 207.0951. $C_8H_{17}NO_3S$ requires M, 207.0929.

(ii) Preparation of 2,3-Dihydro-4H-1,4-thiazin-3-one

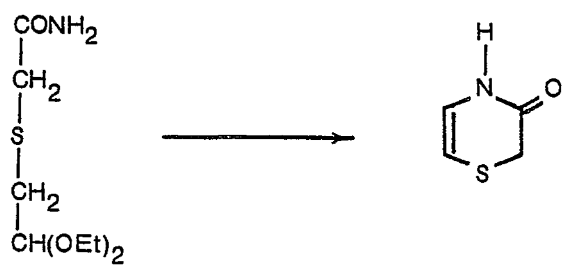

(15) (16)

The amide (15) (4g) was dissolved in benzene (80ml), *p*-toluenesulphonic acid (0.01g) added, and the mixture heated under reflux in an inert atmosphere with a Dean and Stark trap for 3 hours. Removal of solvent and chromatography on silica eluting with pet. ether-ethyl acetate mixtures gave the thiazine (16) as white plates m.p. 74.5—75° (ex ethyl acetate- pet.ether) having $v_{max}$ ($CHCl_3$) 3200, 1690, 1635, 1625, 1380 and 690$cm^{-1}$, $\delta$ ($CDCl_3$) 3.28 (2H, s, $CH_2$), 5.57 (1H, d, J 7Hz, =CH—S), 6.37 (1H, dd, J 5,7 Hz, =CH—N), and 9.13 (1H, br, N—H), $\lambda_{max}$ (EtOH), 230 ($\varepsilon$, 2940) and 302 ($\varepsilon$, 2690)nm. (*Found:* C, 41.4; H, 4.4; N, 12.1. $C_4H_5NSO$ requires C, 41.7; H, 4.35; N, 12.2%).

(iii) Preparation of Sodium Z-2-acetamido-1-ethenylthiolate

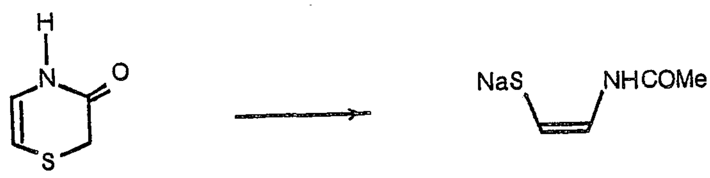

(16) (7)

The thiazine (16) (0.575 g) was dissolved in liquid $NH_3$ (20 ml) and to this solution, under argon and with stirring, was added sodium metal (0.23 g). When the blue colour had disappeared, ammonium chloride (0.2675 g) was added. The ammonia was then allowed to evaporate and the last traces removed under vacuum at 80°. This gave the sodium thiolate (7) as a buff coloured solid.

Example 6

Lithium Z-2-acetamido-1-ethenylthiolate

The thiazine (16) (0.575 g) was dissolved in liquid $NH_3$ (20 ml) and to this solution, under argon, with stirring, was added lithium metal (0.07 g). When the blue colour had disappeared, ammonium chloride (0.2675 g) was added. The ammonia was then allowed to evaporate and the last traces removed under vacuum to give the lithium thiolate as a white solid.

**Claims**

1. A salt of a compound of the formula (V):

$$H—S—CR_2{=}CR_3R_4$$

wherein $R_2$ and $R_3$ are each hydrogen atoms and $R_4$ is a group $NHCO_nR_6$ wherein n is 1 or 2, and $R_6$ is a lower alkyl group, a phenyl group or a phenyl group substituted by one or two halogen atoms, lower alkyl or lower alkoxy groups.

2. A sodium, thallium, silver or lithium salt as claimed in claim 1, wherein n is 1 and $R_6$ is methyl.
3. Silver Z-2-acetamido-1-ethenylthiolate.
4. Silver E-2-acetamido-1-ethenylthiolate.
5. Silver Z-2-methoxycarbonylamino-1-ethenylthiolate.
6. Sodium Z-2-acetamido-1-ethenylthiolate.
7. Lithium Z-2-acetamido-1-ethenylthiolate.

**Revendications**

1. Sel d'un composé de formule (V):

$$H—S—CR_2{=}CR_3R_4$$

dans laquelle $R_2$ et $R_3$ sont chacun des atomes d'hydrogène et $R_4$ est un groupe $NHCO_nR_6$, où n est égal à 1 ou 2 et $R_6$ est un groupe alcoyle inférieur, un groupe phényle ou un groupe phényle substitué par un ou deux atomes d'halogène, des groupes alcoyle inférieur ou alcoxy inférieur.

2. Sel de sodium, de thallium, d'argent ou de lithium suivant la revendication 1, dans lequel n est égal à 1 et $R_6$ est un groupe méthyle.
3. Z-2-Acétamido-1-éthénylthiolate d'argent.
4. E-2-Acétamido-1-éthénylthiolate d'argent.
5. Z-2-Méthoxycarbonylamino-1-éthénylthiolate d'argent.
6. Z-2-Acétamido-1-éthénylthiolate de sodium.
7. Z-2-Acétamido-1-éthénylthiolate de lithium.

**Patentansprüche**

1. Ein Salz einer Verbindung der Formel (V):

$$H—S—CR_2{=}CR_3R_4$$

in welcher $R_2$ und $R_3$ jeweils Wasserstoffatome sind und $R_4$ eine Gruppe $NHCO_nR_6$ ist, wobei n 1 oder 2 ist, und $R_6$ eine niedere Alkylgruppe, eine Phenylgruppe oder eine durch ein oder zwei Halogenatome, niedere Alkyl- oder Alkoxygruppen substituierte Phenylgruppe ist.

2. Ein Natrium-, Thallium-, Silber- oder Lithiumsalz wie in Anspruch 1 beansprucht, in welchem n 1 und $R_6$ Methyl ist.

3. Silber-Z-2-acetamido-1-äthenylthiolat.

4. Silber-E-2-acetamido-1-äthenylthiolat.

5. Silber-Z-2-methoxycarbonylamino-1-äthenylthiolat.

6. Natrium-Z-2-acetamido-1-äthenylthiolat.

7. Lithium-Z-2-acetamido-1-äthenylthiolat.